# EUROPEAN PATENT APPLICATION

(11) **EP 1 264 591 A2**
(43) Date of publication of application: **11.12.2002**
(21) Application number: 02253963.9
(22) Date of filing: 06.06.2002
(51) Int. Cl.: A61K 7/06, A61K 7/48

(54) **Cosmetic composition**

(30) Priority: 06.06.2001 JP 2001171268; 26.10.2001 JP 2001329069
(71) Applicant: Ajinomoto Co., Inc., Tokyo 104-8315 (JP)
(72) Inventor: Yamato, Nayoa, c/o Aminoscience, Kawasaki-shi, Kanagawa 210-8681 (JP)
(74) Representative: Nicholls, Kathryn Margaret

(57) **Abstract**

Herein is disclosed a cosmetic composition comprising, as active ingredients, one or more members selected from the group consisting of mono-N^{α}-acylarginines and cosmetic powders whose surfaces have been treated with a mono-N^{α}-acylarginine (Ingredient A) and one or more members selected from the group consisting of polyhydric alcohols (Ingredient B), which cosmetic composition is excellent in conditioning effects such as moist feeling and voluminous feeling in the case of hair cosmetics and excellent in feelings upon use such as smoothening property and moist feeling without sticky feeling and occlusive feeling in the case of skin cosmetics.

## Description

The present invention relates to a cosmetic composition, more particularly to a cosmetic composition comprising, as active ingredients, one or more members selected from the group consisting of mono-N^{α}-acylarginines and cosmetic powders whose surfaces have been treated with a mono-N^{α}-acylarginine (Ingredient A) and one or more members selected from the group consisting of polyhydric alcohols (Ingredient B).

Embodiments of the cosmetic composition of the present invention may provide conditioning effects such as moist feeling and voluminous feeling for the hair and feelings upon use on the skin such as smoothing property and moist feeling without sticky feeling and occlusive feeling.

In a cosmetic composition (hereinafter sometimes referred to simply as "cosmetic"), a polyhydric alcohol is used as a moisturing agent which absorbs moisture whereby moisture, softness and smoothness can be provided to the skin or hair. It is also used for the purpose of preventing a cosmetic product from getting dried or stabilizing the emulsion in the case of emulsion-based cosmetics.

As other moisturing agents, there may be mentioned a hydrophilic matter occurring in horny substance called NMF (Natural Moisturing Factor), saccharides and the like. The hydrophilic matter is, however, accompanied by the problems that it may drastically change the viscosity of cosmetics and that it may destroy the emulsion in the case of emulsion-based cosmetics. In the sensory aspect, both the hydrophilic matter and saccharides provide strong sticky feeling and occlusive feeling to the skin and hair, and therefore, are poor in feelings upon use.

In hair cosmetics, a polyhydric alcohol is used for the purposes of preventing the hair after washed from getting dried and providing moist feeling to the hair. However, such polyhydric alcohol must be incorporated into such cosmetics in a large amount to get sufficient moist feeling, which causes, in turn, sticky feeling and insufficient voluminous feeling to the hair after dried. Therefore, it is poor in feelings upon use.

In skin cosmetics, a polyhydric alcohol is incorporated for the purpose of providing moist feeling to the skin after applied. However, such skin cosmetics are not sufficiently excellent in feelings upon use, since it lowers smoothening property upon application of a skin cosmetics or causes sticky feeling and occlusive feeling to the skin when incorporated in large amounts.

In Japanese Patent Application Laid-Open (Kokai) No. 228348/1999 is disclosed a cosmetic composition comprising one or more members selected from mono-N^{α}-long chain-acylarginines or a powder whose surface has been treated with a mono-N^{α}-long chain-acylarginine, which is excellent in providing conditioning effects such as moist feeling and settlement of the hair and also excellent in touch feeling such as non-strained feeling for the skin. However, satisfactory moist feeling for the hair and skin cannot be attained by the composition of the invention alone, and thus the composition is not sufficiently excellent in feelings upon use.

For the purpose of enhancing spreadability or smoothening property upon application of a skin cosmetic, N^{ε}-lauroyl-L-lysine is sometimes incorporated. However, feelings upon use are not sufficiently excellent since satisfactory moist feeling is not attained for the skin.

It is thus desirable that the present invention provide a cosmetic composition embodiments of which may provide for the hair conditioning effects such as moist feeling and voluminous feeling upon the hair being washed and dried, as well as feelings upon use on the skin such as smoothing property, moist feeling and the like, without sticky feeling and occlusive feeling.

The present inventor has found that concurrent use or the use in combination of one or more members selected from the group consisting of mono-N^{α}-acylarginines and cosmetic powders whose surfaces have been treated with a mono-N^{α}-acylarginine and one or more members selected from the group consisting of polyhydric alcohols may be advantageous in these respects.

Accordingly, the present invention relates to a cosmetic composition comprising, as active ingredients, one or more members selected from the group consisting of mono-N^{α}-acylarginines and cosmetic powders whose surfaces have been treated with a mono-N^{α}-acylarginine (Ingredient A) and one or more members selected from the group consisting of polyhydric alcohols (Ingredient B).

The present invention relates also to such cosmetic composition as mentioned above, wherein said mono-N^{α}-acylarginine is represented by the following general formula (1): 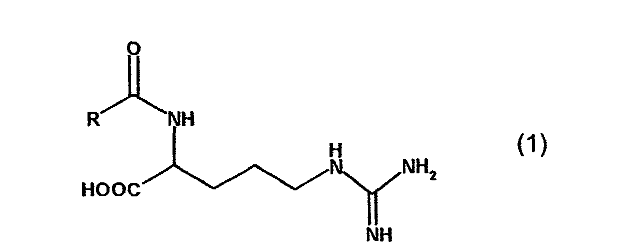 wherein R represents a straight-chain or branched-chain alkyl or alkenyl group having 1-21 carbon atoms.

Moreover, the present invention relates to a cosmetic composition comprising, as active ingredients, one or more members selected from the group consisting of mono-N^{α}-acylarginine crystals which are obtainable by neutralizing an acidic or basic solution of a mixed solvent composed of water and one or more members selected from the group consisting of lower alcohols and polyhydric alcohols, in which mixed solvent a mono-N^{α}-acylarginine has been dissolved, whereby the mono-N^{α}-acylarginine is crystallized, and surface-treated cosmetic powders whose surfaces have been treated with mono-N^{α}-acylarginine crystals (Ingredient A') and one or more members selected from the group consisting of polyhydric alcohols (Ingredient B).

Still moreover, the present invention relates to a cosmetic composition such as a hair cosmetic composition, a hair detergent composition, a skin cosmetic composition, or the like, which cosmetic composition comprises, as active ingredients, one or more members selected from the group consisting of mono-N^{α}-acylarginines and crystals thereof, and cosmetic powders whose surfaces have been treated with a mono-N^{α}-acylarginine and/or crystals thereof (Ingredient A'') and one or more members selected from the group consisting of polyhydric alcohols (Ingredient B).

Further, the present invention relates to a cosmetic composition such as a hair cosmetic composition, a hair detergent composition, a skin cosmetic composition, or the like, which cosmetic composition comprises, as active ingredients, one or more members selected from the group consisting of mono-N^{α}-acylarginines represented by the following general formula (2) and crystals thereof, and cosmetic powders whose surfaces have been treated therewith (Ingredient A''') and one or more members selected from the group consisting of polyhydric alcohols (Ingredient B). 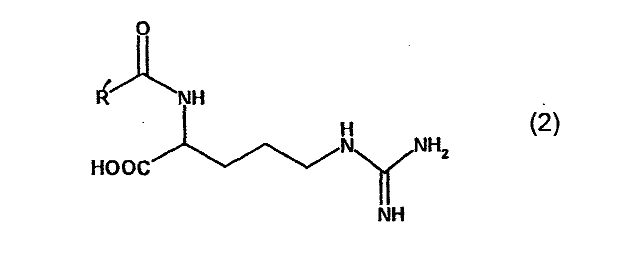 wherein R' represents a straight-chain or branched-chain alkyl or alkenyl group having 11-15 carbon atoms.

Embodiments of the invention are described below, by way of example only, and with reference to the accompanying drawings, of which:
Fig. 1 shows the powder X-ray diffraction pattern of the mono-N^{α}-lauroyl-L-arginine crystals (Compound of Synthetic Example 1).
Fig. 2 (a) and (b) show the infrared absorption spectra (KBr method) of the mono-N^{α}-lauroyl-L-arginine crystals (Compound of Synthetic Example 1).
Fig. 3 shows the powder X-ray diffraction pattern of the mono-N^{α}-myristoyl-L-arginine crystals (Compound of Synthetic Example 2).
Fig. 4 (a) and (b) show the infrared absorption spectra (KBr method) of the mono-N^{α}-myristoyl-L-arginine crystals (Compound of Synthetic Example 2).
Fig. 5 shows the powder X-ray diffraction pattern of the mono-N^{α}-palmitoyl-L-arginine crystals (Compound of Synthetic Example 3).
Fig. 6 (a) and (b) show the infrared absorption spectra (KBr method) of the mono-N^{α}-palmitoyl-L-arginine crystals (Compound of Synthetic Example 3).
Fig. 7 shows the powder X-ray diffraction pattern of the mono-N^{α}-lauroyl-L-arginine (Compound of Synthetic Example 4).
Fig. 8 (a) and (b) show the infrared absorption spectra (KBr method) of the mono-N^{α}-lauroyl-L-arginine (Compound of Synthetic Example 4).

Embodiments of the present invention will be described below in greater detail.

First, Ingredient A of the inventive cosmetic composition will be described.

The mono-N^{α}-acylarginine to be incorporated in the cosmetic composition of the present invention can be obtained by reacting arginine with a long chain fatty acid halide in a hydrophilic solvent under an alkaline condition (around pH 12) as described in Japanese Patent Application Laid-Open (Kokai) No. 23729/1973 or by heat-dehydrating (i.e., dehydrating by heating) the salt of arginine and a fatty acid at a temperature of 100 to 250°C as described in Japanese Patent Application Laid-Open (Kokai) No. 1513/1974. Alternatively, as shown in Japanese Patent Application Laid-Open (Kokai) No. 228527/1999, crystals of mono-N^{α}-acylarginine can be obtained by reacting arginine with a long chain fatty acid halide in a mixed solvent of water and a lower alcohol such as methanol, ethanol, propanol, butanol, isopropanol, or t-butanol and/or a polyhydric alcohol such as ethylene glycol, propylene glycol, 1,3-butylene glycol, isoprene glycol, or glycerin, in the presence of an alkali (pH 7 to 13), and converting the reaction mixture into an acidic or basic aqueous solution using an acid or a base, whereby the whole is dissolved well, followed by adjusting the pH to 5 to 7. In the acylation reaction, in the case that the pH upon acylating is lower than 7 or higher than 13, the reaction yield rapidly decreases.

Moreover, the mono-N^{α}-acylarginine to be incorporated in the cosmetic composition of the present invention may exhibit its effects even if it is in the amorphous state, but the crystals thereof obtainable by the above-described methods are preferable particularly in view of conditioning properties such as moist feeling and voluminous feeling for the hair. By the way, amorphous mono-N^{α}-acylarginine may be prepared by the method described in the above-mentioned Japanese Patent Application Laid-Open (Kokai) No. 23729/1973, for example (cf. Synthetic Example 4 described later on).

The mono-N^{α}-acylarginine to be incorporated in the cosmetic composition of the present invention has a straight-chain or branched-chain saturated or unsaturated fatty acid acyl group having 2 to 22 carbon atoms, and examples thereof include mono-N^{α}-acetylarginine, mono-N^{α}-propionylarginine, mono-N^{α}-2-ethylhexanoylarginine, mono-N^{α}-isostearoylarginine, mono-N^{α}-oleoylarginine, mono-N^{α}-octanoylarginine, mono-N^{α}-decanoylarginine, mono-N^{α}-lauroylarginine, mono-N^{α}-myristoylarginine, mono-N^{α}-palmitoylarginine, mono-N^{α}-stearoylarginine, mono-N^{α}-octyldodecylarginine, mono-N^{α}-behenoylarginine, mono-N^{α}-coconut oil fatty acid acylarginine, mono-N^{α}-palm kernel oil fatty acid acylarginine, mono-N^{α}-beef tallow fatty acid acylarginine, and the like. Of these, mono-N^{α}-decanoylarginine, mono-N^{α}-lauroylarginine, mono-N^{α}-myristoylarginine, mono-N^{α}-palmitoylarginine, mono-N^{α}-stearoylarginine, and mono-N^{α}-coconut oil fatty acid acylarginine are preferable in view of feelings upon use for the skin. On the other hand, mono-N^{α}-lauroylarginine, mono-N^{α}-myristoylarginine, mono-N^{α}-palmitoylarginine, and mono-N^{α}-stearoylarginine are particularly excellent in the ability of imparting moist feeling and voluminous feeling to the hair. Of these, particularly preferred are mono-N^{α}-lauroylarginine, mono-N^{α}-myristoylarginine, and mono-N^{α}-palmitoylarginine. Additionally, arginine may be used in any one of D-form, L-form, and DL-form.

Moreover, either wet treatment or dry treatment may be employed as the surface treatment in the preparation of the cosmetic powder whose surfaces have been treated with a mono-N^{α}-acylarginine (hereinafter, sometimes referred to as "surface-treated powder"), which may be incorporated in the cosmetic composition of the present invention solely or in combination with a mono-N^{α}-acylarginine. In the case of wet treatment, the surface-treated powder can be obtained by charging a cosmetic powder (a powder for cosmetics) into an aqueous solution of a strong acid or a strong alkali or an aqueous solution containing an organic solvent such as a lower alcohol, and also dissolving concurrently a mono-N^{α}-acylarginine therein, followed by neutralizing the mixture. Alternatively, in the case of dry treatment, the surface-treated powder can be obtained by charging a powder to be coated and a mono-N^{α}-acylarginine into a mixing grinder and coating the powder with the mono-N^{α}-acylarginine mechanically at a rotation number of several thousand to several ten thousand r.p.m.

The starting powder (cosmetic powder) usable for the preparation of the surface-treated powder to be incorporated in the cosmetic composition of the present invention is not particularly limited, and examples thereof include organic powders such as nylon powder, polyethylene powder, poly(methyl methacrylate) powder, polystyrene powder, styrene-acrylic acid copolymer resin powder, benzoguanamine resin powder, polytetrafluoroethylene resin powder, cellulose powder, silicone powder and the like; extender pigments such as talc, kaolin, mica, sericite, white mica, phlogopite, synthetic mica, lepidolite, lithia mica, vermiculite, magnesium carbonate, calcium carbonate, aluminum silicate, barium silicate, calcium silicate, magnesium silicate, strontium silicate, tungstic acid metal salt, silica, zeolite, barium sulfate, calcined calcium sulfate, calcium phosphate, fluorine apatite, hydroxyapatite, ceramic powder, zinc myristate, calcium palmitate, aluminum stearate, boron nitride and the like; ultraviolet ray-shielding powders such as titanium dioxide, zinc oxide, iron oxide (red iron oxide), iron titanate, fine powdery titanium oxide, needle-shaped titanium oxide, spindle-shaped titanium oxide, rod-shaped titanium oxide, fine powdery zinc oxide, flake-shaped zinc oxide and the like; white and coloring pigments such as γ-iron oxide, yellow iron oxide, black iron oxide, carbon black, mango violet, palto violet, chromium oxide, cerium oxide, chromium hydroxide, cobalt titanate, ultramarine, Prussian blue, titanium oxide-coated mica, titanium oxide-coated bismuth oxychloride, bismuth oxychloride, aluminum powder, copper powder, the Red No. 201 and the like; natural pigments such as chlorophyll, β-carotene and the like; and the like.

The amount of the mono-N^{α}-acylarginine upon the surface treatment of such a cosmetic powder is preferably from 0.01 to 30 parts by weight, more preferably 0.1 to 20 parts by weight, further preferably from 0.5 to 10 parts by weight, relative to 100 parts by weight of the cosmetic powder to be surface-treated in view of effecting the efficient and uniform surface treatment.

The incoporating amount of a mono-N^{α}-acylarginine and/or a cosmetic powder subjected to the surface treatment with a mono-N^{α}-acylarginine is optionally determined depending on the aimed-at product and is not particularly limited, but it is usually from 0.01 to 50 parts by weight, preferably from 0.05 to 10 parts by weight, more preferably from 0.05 to 5 parts by weight based on the total amount of the cosmetic composition.

With regard to these mono-N^{α}-acylarginines and/or cosmetic powders subjected to the surface treatment with a mono-N^{α}-acylarginine, one member selected from these may be used solely, or two or more members may be used in combination.

Next will be explained a polyhydric alcohol which constitutes Ingredient B of the cosmetic composition of the present invention.

The polyhydric alcohol herein means an alcohol having two or more hydroxyl groups in the molecule, and examples thereof can include glycerol, propylene glycol, 1,3-butylene glycol, isoprene glycol, polyethylene glycol, diglycerol, triglycerol, tetraglycerol, pentaglycerol, hexaglycerol, decaglycerol, sorbitol, ethylene glycol, erythritol and the like.

The incorporating amount of the polyhydric alcohol to be used in the present invention is optionally determined depending on the aimed-at product and is not particularly limited, but it is usually employed in the range of 0.001 to 50% by weight based on the total amount of the cosmetic composition. Particularly preferred is the range of 0.01 to 10% by weight. When the amount is less than 0.001% by weight, the effects of the present invention can sometimes not be sufficiently exhibited and when it exceeds 50% by weight, sticky feeling is sometimes imparted to the skin or hair and therefore, such insufficient amounts and exceeding amounts are both not preferred.

With regard to these polyhydric alcohols, one member selected from them may be employed solely or two or more members in combination.

The mixing ratio (weight ratio) of Ingredient A (or Ingredient A', A'', or A''') to Ingredient B is optionally determined depending on the aimed-at product and is not particularly limited, but it may be usually employed in the range of 100:1 to 1:100 parts by weight (i.e., the mixing ratio of (Ingredient A)/(Ingredient B) of 100 to 0.01). Particularly preferred is the range of 5:1 to 1:100 (i.e., the mixing ratio of 5 to 0.01).

The cosmetic composition of the present invention comprising such Ingredient A and B can be used as hair detergents such as shampoo, rinse, rinse-in-shampoo, conditioning shampoo, and the like; various hair cosmetics including hair lotion, hair conditioner, hair treatment, hear cream, hair spray, hair liquid, hair wax, hair water, hair-styling preparation, perming liquid, hair color, acidic hair color, hair manicure, etc.; or various skin cosmetics such as skin lotion, milky lotion, face wash, makeup remover, cleansing lotion, emollient lotion, nourishing cream, emollient cream, massage cream, cleansing cream, body shampoo, hand soap, bar soap, shaving cream, sunburn cosmetics, deodorant powder, deodorant lotion, deodorant spray, makeup removing gel, moisture gel, moisture essence, UV-preventing essence, shaving foam, face powder, foundation, lipstick, cheek rouge, eyeliner, eye shadow, eyebrow pencil, bathing preparation, etc.; mouth detergent such as toothpaste; and the like.

By the way, in addition to the above Ingredients A and B, any other various ordinary additives may be added to the cosmetic composition of the present invention insofar as they do not interfere with the effects of the present invention. Examples thereof include surfactants (anionic surfactants, cationic surfactants, amphoteric surfactants, nonionic surfactants, etc.), waxes, vegetable oils, animal oils and fats, derivatives of natural oils and fats, mineral oils and fats, lower and higher fatty acid esters, synthetic oils and fats (N-acylglutamic acid esters etc.), polymeric substances, alcohols, polyhydric alcohols, extracts (natural perfume materials etc.), amino acids, nucleic acids, vitamins, hydrolyzed proteins and derivatives thereof, glyceryl oleate, enzymes, anti-inflammatory agents, microbicides, antiseptics, anti-oxidants, UV absorbents, chelating agents, sweat retardants, oxidizing dyes, pH regulators, pearly additives, moisturizers, etc., which are raw materials described in Standards of Cosmetic Materials, Specifications for Ingredients of Different Types of Cosmetics, Specifications for Materials of Quasi-drugs, the Japanese Pharmacopoeia, Official Specifications for Food Additives, and the like.

### EXAMPLES

Embodiments of the present invention will be described in greater detail with reference to the following examples, by way of illustration only.

### <Synthetic Example 1>: Synthesis of mono-N^{α}-lauroyl-L-arginine crystals

To 1,106 g of L-arginine were added 6,919 g of isopropyl alcohol and 2,964 g of water. Thereto were concurrently added dropwise 1,522 g of lauroyl chloride (manufactured by Nippon Oil and Fats Co., Ltd.) and 27 wt% NaOH aqueous solution over a period of 2 hours with the pH being maintained at 10.5 to 11.5 and the reaction temperature being maintained at 10 to 13°C. After 1 hour of aging, the reaction mixture was warmed to 50°C and adjusted to pH 3.8 by adding concentrated hydrochloric acid to dissolve the reaction product completely. Thereafter, the pH was adjusted to 6.0 by adding 27 wt% NaOH aqueous solution to precipitate crystals and the resulting slurry was gradually cooled to 10°C with stirring. When the slurry was cooled to 10°C, the crystals were collected by filtration and washed with 10 kg of water and 4.4 kg of isopropyl alcohol, successively. The resulting washed crystals were dried under reduced pressure to obtain 2,081 g of mono-N^{α}-lauroyl-L-arginine as flake-shaped crystals (yield 92.3%).

The physical properties of the crystals are shown in the following Table 4.

**Table 4**

| | | | | |
|---|---|---|---|---|
| (a) | Decomposition temperature: 159.9°C (DSC peak). | | | |
| (b) | Powder X-ray diffraction peaks (2θ): 12.49, 21.54, 21.85. | | | |
| (c) | Wave numbers of infrared absorption spectra (cm⁻¹): 1683.7, 1654.8, 1625.9, 1544.9, 1471.6, 1461.9, 1446.5, 1407.9. | | | |
| (d) | Elemental analysis (as C₁₈H₃₆O₃N₄): | | | |
| | | C | H | O |
| | Calcd. (%) | 60.64 | 10.17 | 15.72 |
| | Found (%) | 60.5 | 10.2 | 15.6 |

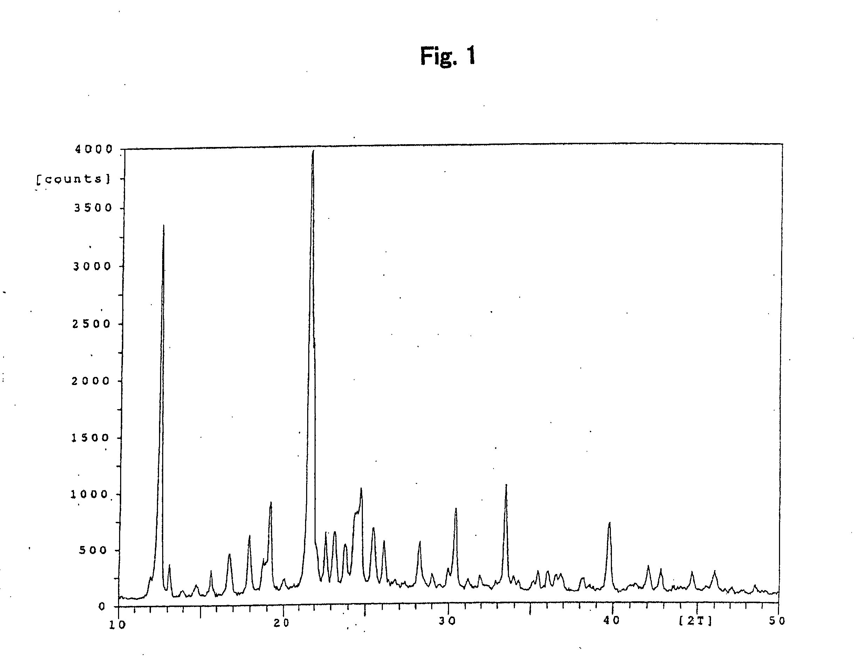

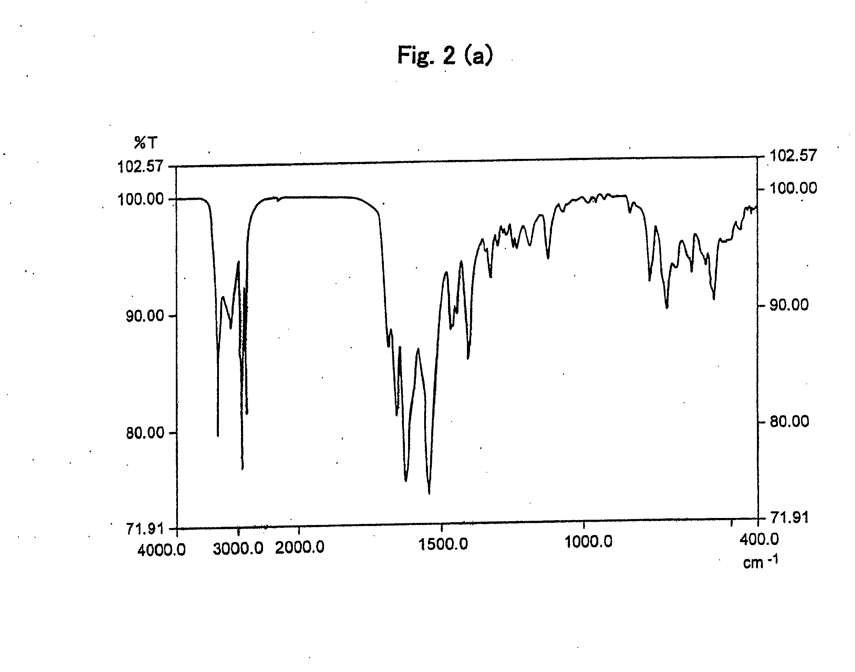

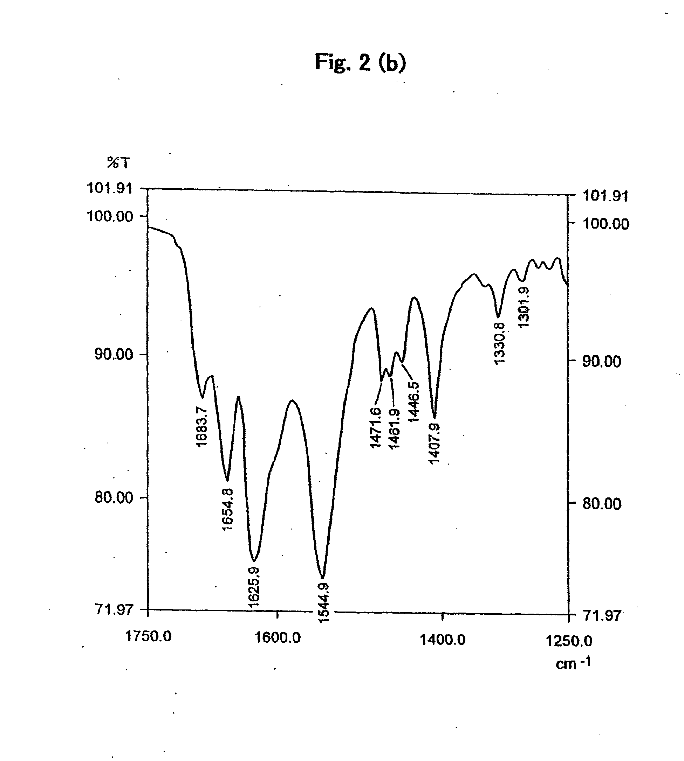

The powder X-ray diffraction pattern of the crystals will be shown in Figure 1, Infrared absorption spectra (KBr method), in Figure 2, and the main peaks of the powder X-ray diffraction, in the following Table 5.

**Table 5**

| 2θ | Strength | 2θ | Strength | 2θ | Strength | 2θ | Strength |
|---|---|---|---|---|---|---|---|
| 11.9 | Weak | 21.5 | Strong | 29.0 | Weak | 36.9 | Weak |
| 12.5 | Strong | 21.9 | Strong | 29.9 | Weak | 38.1 | Weak |
| 13.1 | Weak | 22.6 | Medium | 30.3 | Medium | 39.7 | Medium |
| 14.7 | Weak | 23.2 | Medium | 31.2 | Weak | 42.0 | Weak |
| 15.7 | Weak | 23.8 | Medium | 31.9 | Weak | 42.8 | Weak |
| 16.8 | Medium | 24.3 | Medium | 33.4 | Strong | 44.6 | Weak |
| 17.9 | Medium | 24.7 | Medium | 33.9 | Weak | 45.9 | Weak |
| 18.7 | Weak | 25.4 | Medium | 35.4 | Weak | | |
| 19.2 | Medium | 26.1 | Medium | 36.0 | Weak | | |
| 20.0 | Medium | 28.2 | Medium | 36.5 | Weak | | |

### <Synthetic Example 2>: Synthesis of mono-N^{α}-myristoyl-L-arginine crystals

As in Synthetic Example 1, to 113 g of L-arginine were added 706 g of isopropyl alcohol and 302 g of water. Thereto were concurrently added dropwise 176 g of myristoyl chloride (manufactured by Nippon Oil and Fats Co., Ltd.) and 27 wt% NaOH aqueous solution over a period of 2 hours with the pH being maintained at 10.5 to 11.5 and the reaction temperature, at 10 to 13°C. After 1 hour of aging, the reaction mixture was warmed to 50°C and adjusted to below pH 2.7 by adding concentrated hydrochloric acid to dissolve the reaction product completely. Thereafter, the pH was adjusted to 6.0 by adding 27 wt% NaOH aqueous solution to precipitate crystals and the resulting slurry was gradually cooled to 10°C with stirring. When the slurry was cooled to 10°C, the crystals were collected by filtration and washed with 1,500 g of water and 492 g of isopropyl alcohol, successively. The resulting washed crystals were dried under reduced pressure to obtain 230 g of mono-N^{α}-myristoyl-L-arginine as flake-shaped crystals (yield 92.2%).

The physical properties of the crystals are shown in the following Table 6.

**Table 6**

| | |
|---|---|
| (a) | Decomposition temperature: 156.2°C (DSC peak). |
| (b) | Powder X-ray diffraction peaks (2θ): 11.575, 14.490, 21.700, 31.020, 33.830. |
| (c) | Wave numbers of infrared absorption spectra (cm⁻¹): 1683.7, 1654.8, 1625.9, 1542.9, 1471.6, 1460.0, 1446.5, 1407.9. |

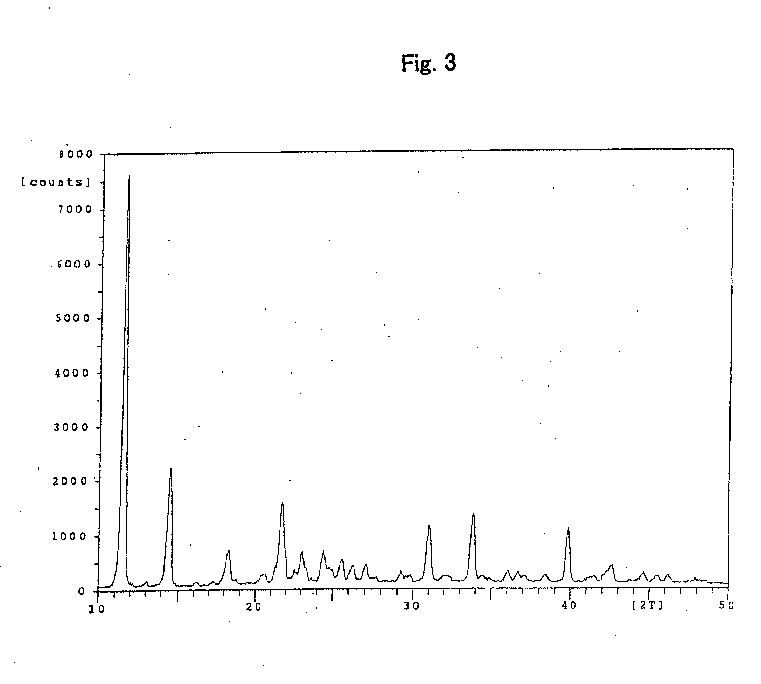

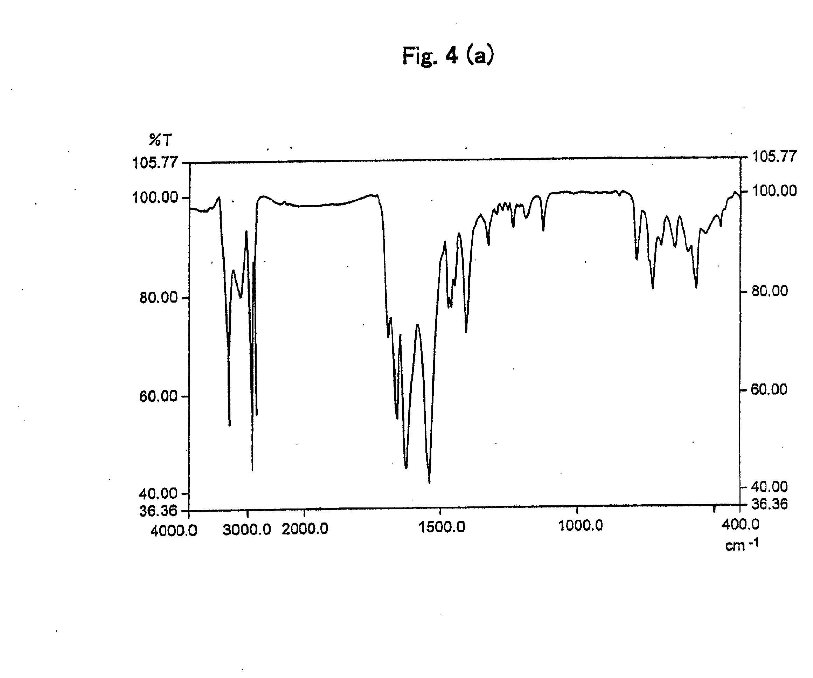

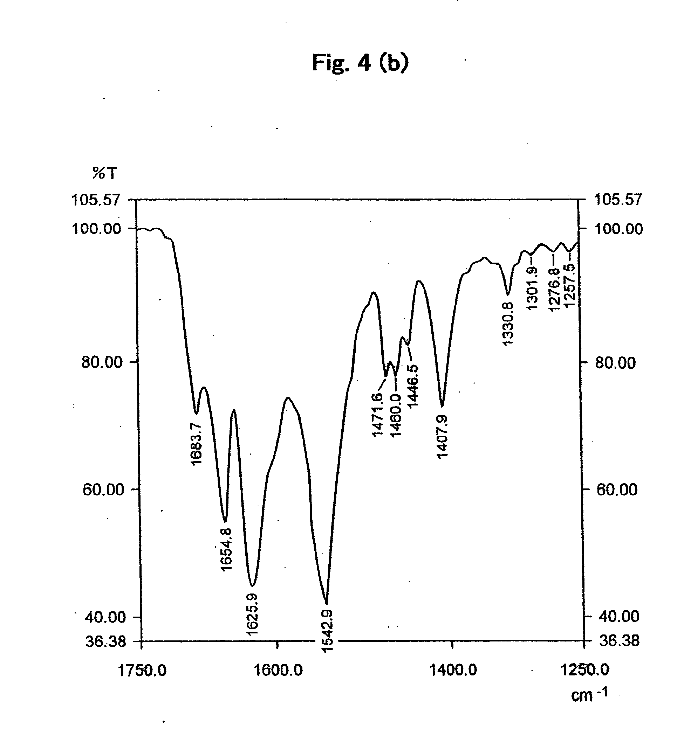

The powder X-ray diffraction pattern of the crystals will be shown in Figure 3, Infrared absorption spectra (KBr method), in Figure 4, and the main peaks of the powder X-ray diffraction, in the following Table 7.

**Table 7**

| 2θ | Strength | 2θ | Strength | 2θ | Strength | 2θ | Strength |
|---|---|---|---|---|---|---|---|
| 11.6 | Strong | 24.4 | Medium | 32.1 | Weak | 41.5 | Weak |
| 13.1 | Weak | 25.6 | Medium | 33.8 | Strong | 42.6 | Medium |
| 14.5 | Strong | 26.3 | Medium | 34.5 | Weak | 44.6 | Weak |
| 18.3 | Medium | 27.1 | Medium | 36.0 | Weak | 45.5 | Weak |
| 18.8 | Weak | 27.7 | Weak | 36.7 | Weak | 46.2 | Weak |
| 20.6 | Weak | 29.3 | Weak | 37.1 | Weak | | |
| 21.7 | Strong | 29.8 | Weak | 38.4 | Weak | | |
| 23.0 | Medium | 31.0 | Strong | 39.9 | Medium | | |

### <Synthetic Example 3>: Synthesis of mono-N^{α}-palmitoyl-L-arginine crystals

As in Synthetic Example 1, to 84.7 g of L-arginine were added 706 g of isopropyl alcohol and 302 g of water. Thereto were concurrently added dropwise 147 g of palmitoyl chloride (manufactured by Aldrich) and 27 wt% NaOH aqueous solution over a period of 2 hours with the pH being at 10.5 to 11.5 and the reaction temperature, at 27 to 30°C. After 1 hour of aging, the reaction mixture was warmed to 50°C and adjusted to below pH 1.8 by adding concentrated hydrochloric acid to dissolve the reaction product completely. Thereafter, the pH was adjusted to 6.0 by adding 27 wt% NaOH aqueous solution to precipitate crystals and the resulting slurry was gradually cooled to 10°C with stirring. When the slurry was cooled to 10°C, the crystals were collected by filtration and washed with 1,200 g of water and 320 g of isopropyl alcohol, successively. The resulting washed crystals were dried under reduced pressure to obtain 190 g of mono-N^{α}-palmitoyl-L-arginine as flake-shaped crystals (yield 94.7%).

The physical properties of the crystals are shown in the following Table 8.

**Table 8**

| | |
|---|---|
| (a) | Decomposition temperature: 151.5°C (DSC peak). |
| (b) | Powder X-ray diffraction peaks (2θ): 10.650, 13.340, 21.590. |
| (c) | Wave numbers of infrared absorption spectra (cm⁻¹): 1683.7, 1654.8, 1625.9, 1542.9, 1471.6, 1460.0, 1448.4, 1407.9. |

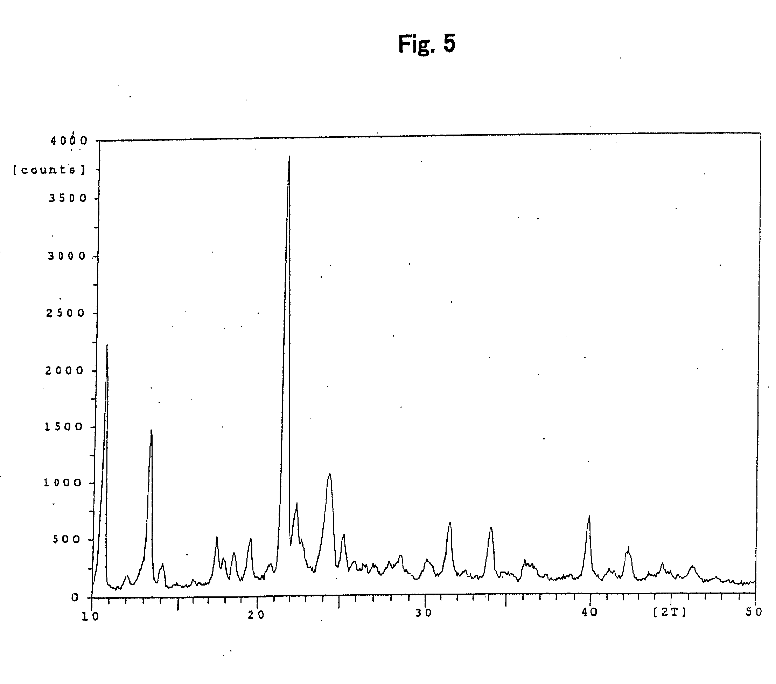

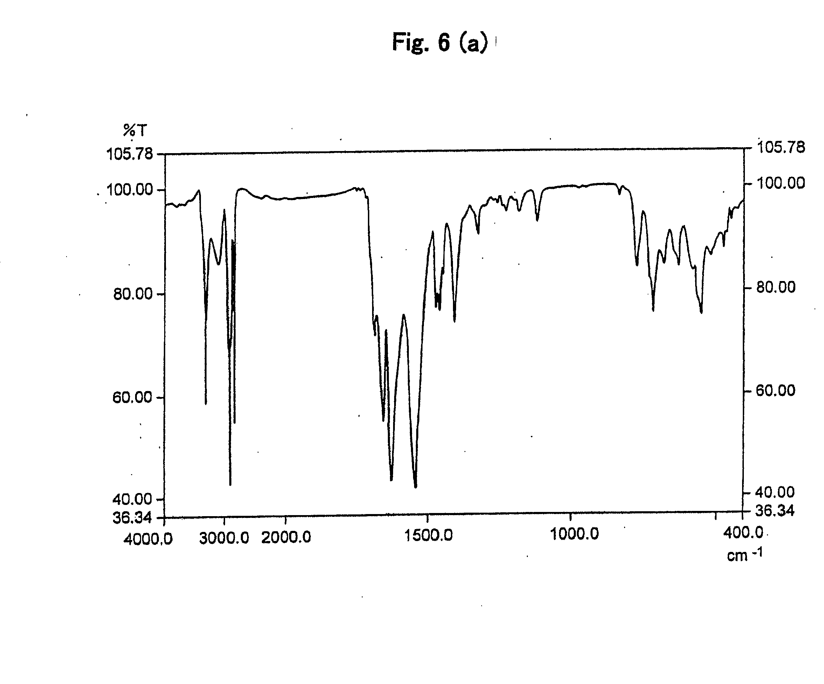

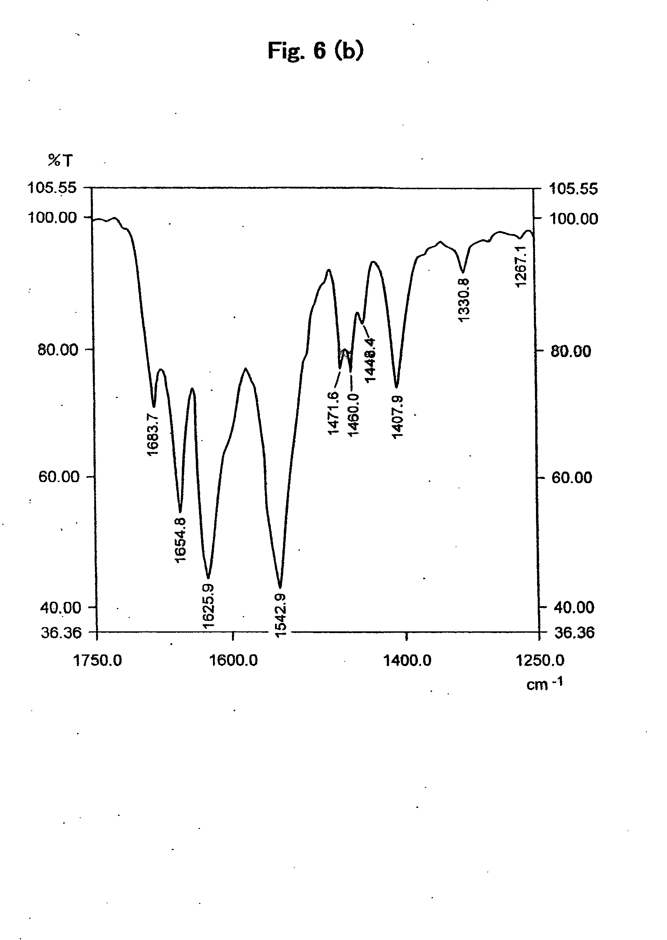

The powder X-ray diffraction pattern of the crystals will be shown in Figure 5, Infrared absorption spectra (KBr method), in Figure 6, and the main peaks of the powder X-ray diffraction, in the following Table 9.

**Table 9**

| 2θ | Strength | 2θ | Strength | 2θ | Strength | 2θ | Strength |
|---|---|---|---|---|---|---|---|
| 10.7 | Strong | 20.8 | Weak | 27.0 | Weak | 36.6 | Weak |
| 12.1 | Weak | 21.6 | Strong | 27.9 | Weak | 39.8 | Medium |
| 13.3 | Strong | 22.4 | Medium | 28.5 | Weak | 42.2 | Weak |
| 14.1 | Weak | 22.8 | Medium | 30.1 | Weak | 44.4 | Weak |
| 17.4 | Medium | 24.3 | Medium | 31.5 | Medium | 46.3 | Weak |
| 17.9 | Weak | 25.2 | Medium | 32.5 | Weak | | |
| 18.6 | Weak | 25.9 | Weak | 34.1 | Medium | | |
| 19.5 | Medium | 26.4 | Weak | 36.0 | Weak | | |

### <Synthetic Example 4>: Synthesis of mono-N^{α}-lauroyl-L-arginine (amorphous)

In accordance with the method of Example 1 in the above-mentioned Japanese Patent Application Laid-Open (Kokai) No. 23729/1973, mono-N^{α}-lauroyl-L-arginine was synthesized.

To 35.0 g of L-arginine were added 118.7 g of acetone and 200 g of water. Thereto were concurrently added dropwise 36.5 g of lauroyl chloride ("lauroyl chloride" manufactured by Nippon Oil and Fats Co., Ltd.) and 45 ml of 8N NaOH aqueous solution over a period of 2 hours with the pH being maintained at 11.5 to 12.0 and the reaction temperature being maintained at 15 to 25°C. After 2 hours of aging, the reaction mixture was adjusted to pH 5.0 with 6N sulfuric acid with cooling and then poured into about 300 ml of ice-water to precipitate crude crystals of mono-N^{α}-lauroyl-L-arginine, which were collected by filtration and then dried (yield 55 g) . The crude crystals were washed by stirring in 300 ml of petroleum benzine and then filtered, and the crystals were dried under reduced pressure to obtain 51 g of mono-N^{α}-lauroyl-L-arginine (yield 85%).

The physical properties of the crystals are shown in the following Table 10.

**Table 10**

| | | | | |
|---|---|---|---|---|
| (a) | Decomposition temperature: 116.6°C (DSC peak). | | | |
| (b) | Powder X-ray diffraction peaks (2θ): 21.56 | | | |
| (c) | Wave numbers of infrared absorption spectra (cm⁻¹): 1653.2, 1630.0, 1543.2, 1471.9, 1462.2, 1448.7, 1408.2. | | | |
| (d) | Elemental analysis (as C₁₈H₃₆O₃N₄): | | | |

| | | C | H | O |
|---|---|---|---|---|
| | Calcd.(%) | 60.64 | 10.17 | 15.72 |
| | Found (%) | 59.72 | 10.31 | 15.04 |

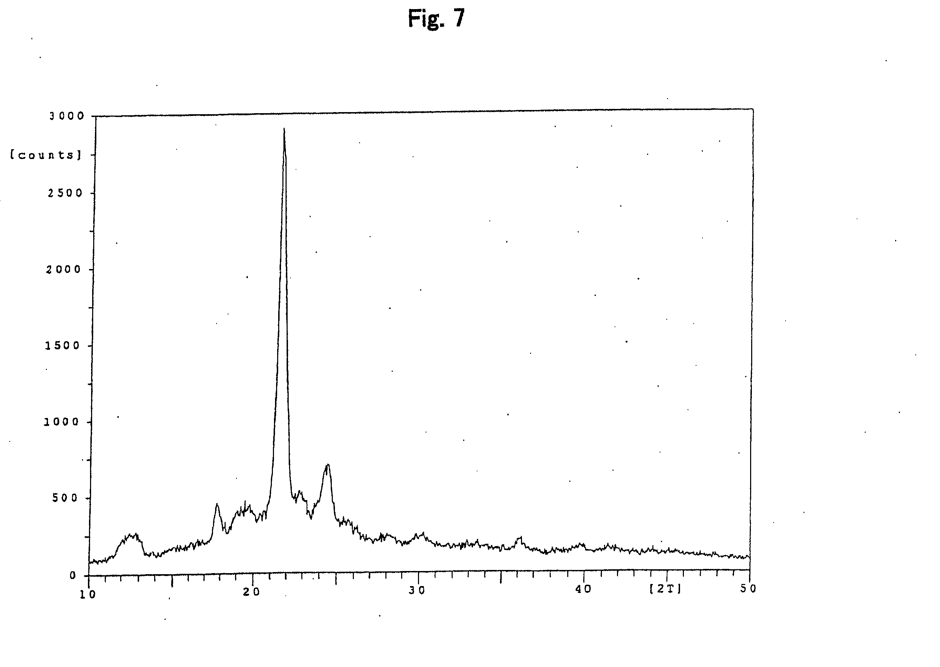

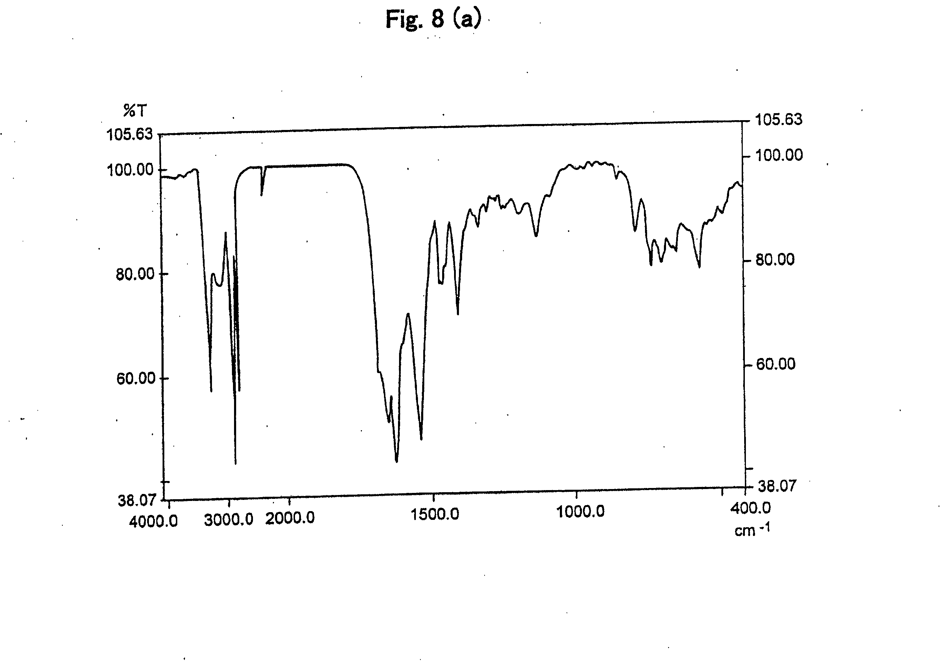

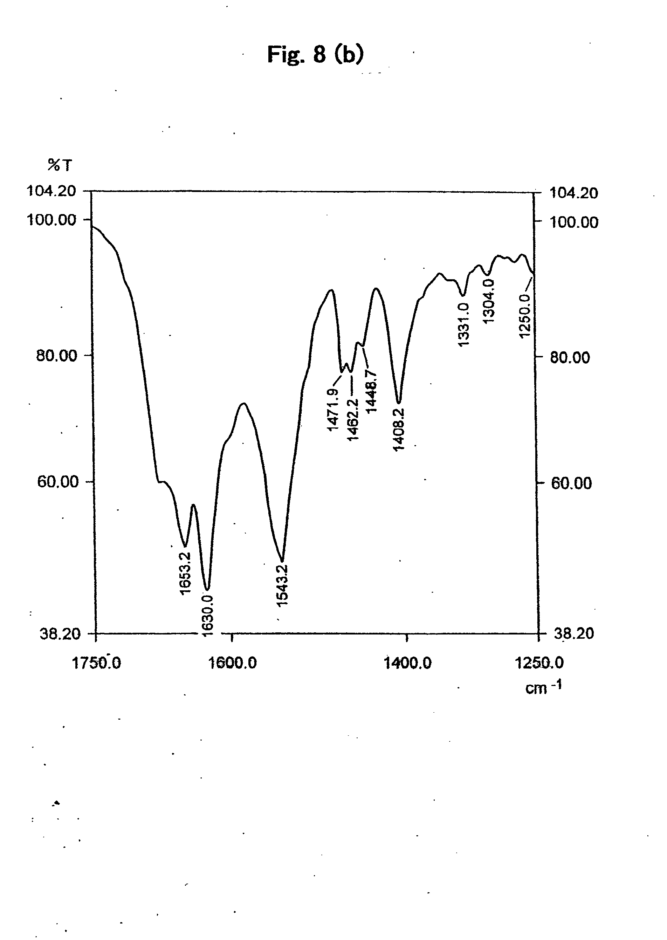

The powder X-ray diffraction pattern thereof will be shown in Figure 7, Infrared absorption spectra (KBr method), in Figure 8, and the main peaks of the powder X-ray diffraction, in the following Table 11.

**Table 11**

| 2θ | Strength | 2θ | Strength | 2θ | Strength |
|---|---|---|---|---|---|
| 13.0 | Weak | 19.0 | Weak | 22.9 | Weak |
| 17.8 | Weak | 21.6 | Strong | 24.5 | Weak |

Characteristic peaks as shown in the case of the mono-N^{α}-lauroyl-L-arginine obtained in Synthetic Example 1 were not observed in the powder X-ray diffraction chart of the mono-N^{α}-lauroyl-L-arginine obtained in the present Synthetic Example and, in this sense, the present compound can be said to be amorphous.

By the way, mono-N^{α}-decanoylarginine, mono-N^{α}-stearoylarginine, and mono-N^{α}-coconut oil fatty acid acylarginine were also produced under almost the same reaction conditions.

### <Surface-treatment Example 1>: Surface treatment of talc with N^{α}-lauroyl-L-arginine

A surface-treated powder was obtained by conducting surface treatment by mixing 5.0 g of talc as a cosmetic powder ("Microace P-30", manufactured by Nippon Talc Co., Ltd.) and 0.25 g of the compound obtained in Synthetic Example 1 and mixing them with stirring for 1 minute twice using a home mixer ("IFM-150" manufactured by Iwatani Sangyo K.K.).

### <Test Example 1>: Shampoo

Shampoos having each composition (expressed by wt%, 100% in total) shown in the following Table 13 were prepared in the usual manner (four kinds of Examples and two kinds of Comparative Example). These shampoos were each applied to a bundle of the hair which had been washed with 1% aqueous solution of sodium lauryl ether sulfate, and then the hair was thoroughly washed with water. After drying, a sensory evaluation was conducted by a panel of five expert panelists with respect to (a) moist feeling of the hair and (b) voluminous feeling of the hair. The evaluation was carried out through the steps of calculating an average value on the basis of the evaluation standard shown in the following Table 12 and rating the case of the average value of 4 or higher as very good (ⓞ), the case of the value of 3.5 to 3.9 as good (○), the case of the value of 3 to 3.5 as moderate (Δ), and the case of the value of 2.9 or lower as bad (×). The evaluation results are also shown in Table 13.

**Table 12:**

| <Evaluation Standard> | | |
|---|---|---|
| (a) Moist feeling after drying | | |
| | 5 | Strong moist feeling |
| | 4 | Moist feeling |
| | 3 | Moderate |
| | 2 | Weak Moist feeling |
| | 1 | No moist feeling |

| (b) Voluminous feeling after drying | | |
|---|---|---|
| | 5 | Strong voluminous feeling |
| | 4 | Voluminous feeling |
| | 3 | Moderate |
| | 2 | Weak voluminous feeling |
| | 1 | No voluminous feeling |

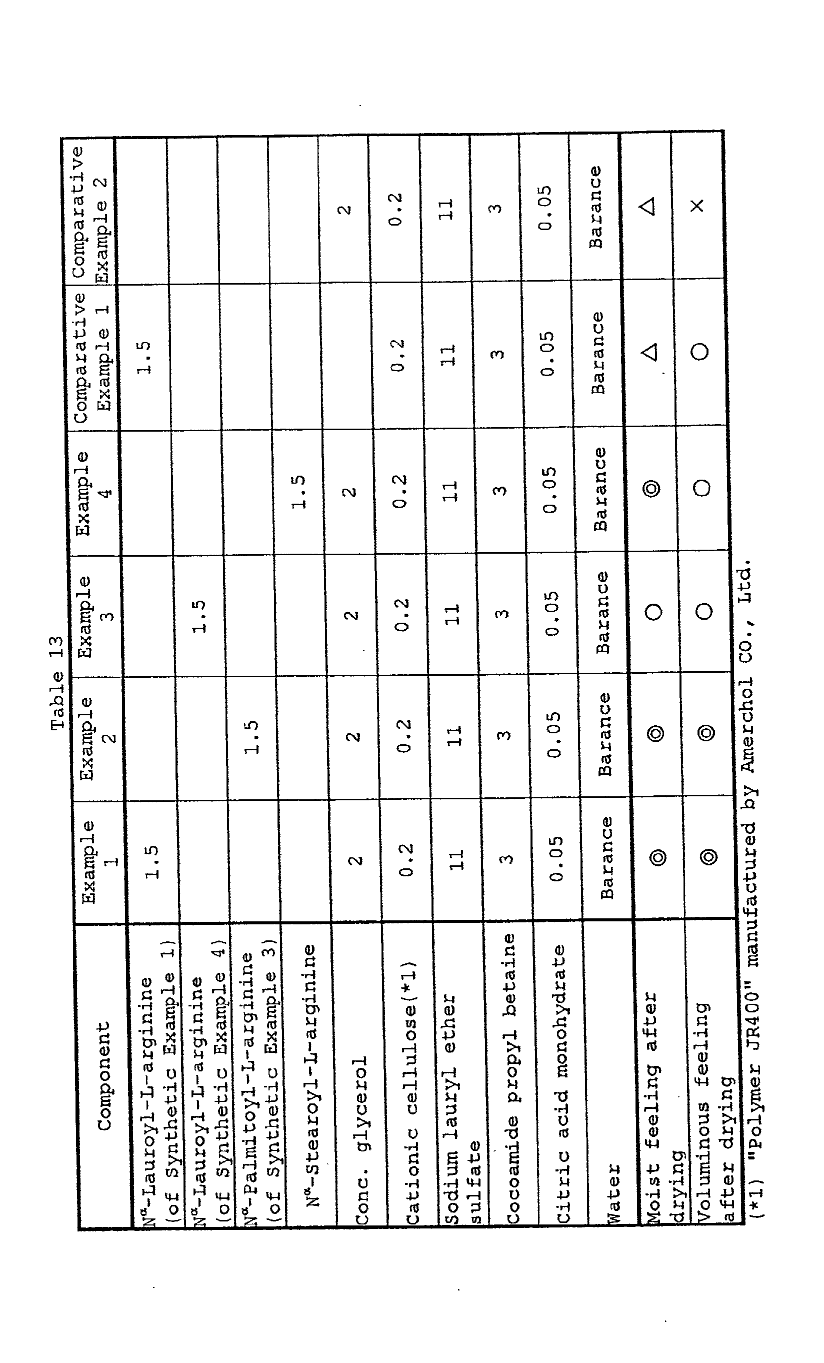

### <Test Example 2>: Milky lotion

Milky lotions having each composition shown in the following Table 15 (expressed by wt%, 100% in total) were prepared (four kinds of Examples and two kinds of Comparative Examples). A suitable amount of each milky lotion was applied to the back of hands. A sensory evaluation was conducted by a panel of five expert panelists with respect to (a) smoothening property upon use, (b) moist feeling after use, (c) sticky feeling after use, and (d) occlusive feeling after use. The evaluation was carried out through the steps of calculating an average value on the basis of the evaluation standard shown in the following Table 14 and rating the case of the average value of 4 or higher as very good (ⓞ), the case of the value of 3.5 to 3.9 as good (○), the case of the value of 3 to 3.5 as moderate (Δ), and the case of the value of 2.9 or lower as bad (×). The evaluation results are also shown in Table 15.

**Table 14:**

| <Evaluation Standard> | |
|---|---|
| (a) Smoothening property after use | |
| 5 | Smoothening |
| 4 | Slight smoothening |
| 3 | Moderate |
| 2 | Weak smoothening |
| 1 | No smoothening |

| (b) Moist feeling after use | |
|---|---|
| 5 | Strong moist feeling |
| 4 | Moist feeling |
| 3 | Moderate |
| 2 | Weak Moist feeling |
| 1 | No moist feeling |

| (c) Sticky feeling after use | |
|---|---|
| 5 | No sticky feeling |
| 4 | Weak sticky feeling |
| 3 | Moderate |
| 2 | Slight sticky feeling |
| 1 | Sticky feeling |

| (d) Occlusive feeling after use | |
|---|---|
| 5 | No occlusive feeling |
| 4 | Weak occlusive feeling |
| 3 | Moderate |
| 2 | Slight occlusive feeling |
| 1 | Occlusive feeling |

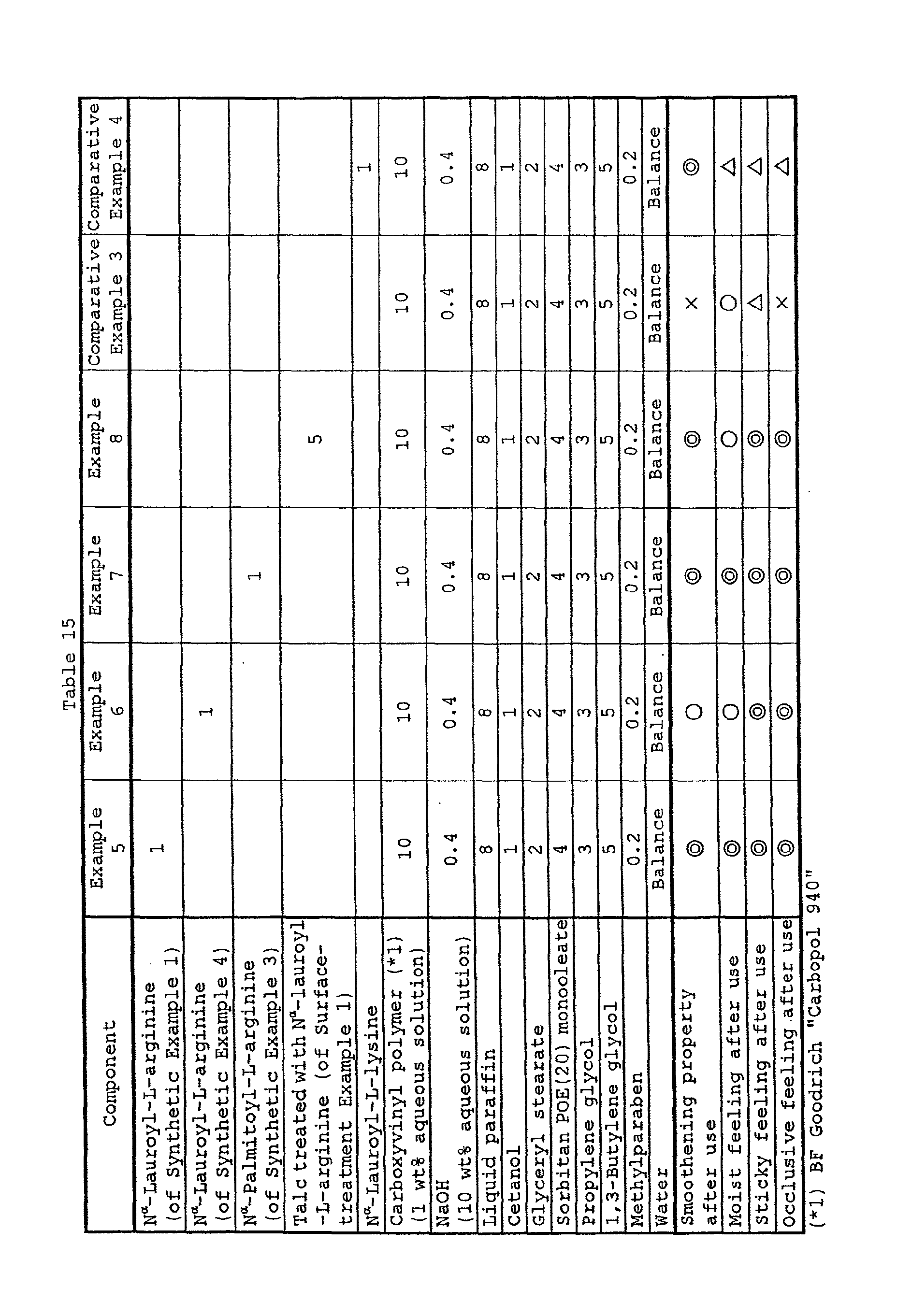

There will be given below Preparation Examples of cosmetic compositions of the present invention.

### <Preparation of Shampoo>

Three kinds of shampoos having each formulation shown in the following Table 16 were prepared in the usual manner.

**Table 16:**

| Shampoo | | | |
|---|---|---|---|
| Ingredient | Example 9 | Example 10 | Example 11 |
| Ingredient A of the invention | | | |
| N^{α}-Lauroyl-L-arginine (of Synthetic Example 1) | 1 | | |
| N^{α}-Cocoyl-L-arginine | | 1 | |
| N^{α}-Palmitoyl-L-arginine (of Synthetic Example 3) | | | 1 |

| Ingredient B of the invention | | | |
|---|---|---|---|
| Conc. glycerin | 2 | | 2 |
| 1,3-Butylene glycol | | 2 | |

| Other ingredients | | | |
|---|---|---|---|
| Sodium lauryl ether sulfate | 10 | 10 | 10 |
| Coconut oil fatty acid diethanolamide | 3 | 3 | 3 |
| Cationic cellulose (*1) | 0.1 | 0.1 | 0.1 |
| Dimethyldiallylammonium chloride-acrylamide copolymer (*2) | 0.3 | 0.3 | 0.3 |
| Antiseptic | Suitable amount | Suitable amount | Suitable amount |
| Water | Balance | Balance | Balance |

| | | | |
|---|---|---|---|
| (*1) "Leoguard GP" manufactured by Lion Corp. | | | |
| (*2) "Lipoflow MN" manufactured by Lion Corp. | | | |

All the shampoos prepared were excellent in conditioning effects such as moist feeling and voluminous feeling after drying for the hair.

### <Preparation of Hair-treatment>

Three kinds of Hair treatment having each formulation shown in the following Table 17 were prepared in the usual manner.

**Table 17:**

| Hair treatment | | | |
|---|---|---|---|
| Ingredient | Example 12 | Example 13 | Example 14 |
| Ingredient A of the invention | | | |
| N^{α}-Lauroyl-L-arginine (of Synthetic Example 1) | 0.3 | | |
| N^{α}-Cocoyl-L-arginine | | 0.3 | |
| N^{α}-Palmitoyl-L-arginine (of Synthetic Example 3) | | | 0.3 |

| Ingredient B of the invention | | | |
|---|---|---|---|
| Glycerol | 3.0 | | |
| 1,3-Butylene glycol | | 3.0 | |
| Sorbitol | | | 3.0 |

| Other ingredients | | | |
|---|---|---|---|
| Silicone oil | 3.0 | 3.0 | 3.0 |
| Liquid paraffin | 1.0 | 1.0 | 1.0 |
| Cetyl alcohol | 1.5 | 1.5 | 1.5 |
| Stearyl alcohol | 1.0 | 1.0 | 1.0 |
| Stearyltrimethyl ammonium chloride | 0.7 | 0.7 | 0.7 |
| Antiseptic | Suitable amount | Suitable amount | Suitable amount |
| Water | Balance | Balance | Balance |

All the hair treatments prepared were excellent in conditioning effects such as moist feeling and voluminous feeling after drying for the hair.

### <Preparation of Hair-styling gel>

Three kinds of hair-styling gel having each formulation shown in the following Table 18 were prepared in the usual manner.

**Table 18:**

| Hair-styling gel | | | |
|---|---|---|---|
| Ingredient | Example 15 | Example 16 | Example 17 |
| Ingredient A of the invention | | | |
| N^{α}-Lauroyl-L-arginine (of Synthetic Example 1) | 1 | | |
| N^{α}-Cocoyl-L-arginine | | 1 | |
| N^{α}-Palmitoyl-L-arginine (of Synthetic Example 3) | | | 1 |

| Ingredient B of the invention | | | |
|---|---|---|---|
| Glycerol | 5 | 5 | 5 |

| Other ingredients | | | |
|---|---|---|---|
| Liquid paraffin | 15 | 15 | 15 |
| Vaseline | 15 | 15 | 15 |
| Bleached beeswax | 2 | 2 | 2 |
| Carboxyvinyl polymer (*1) | 0.1 | 0.1 | 0.1 |
| Xanthan gum | 0.1 | 0.1 | 0.1 |
| Polyoxyethylene hardened castor oil | 3 | 3 | 3 |
| Sodium hydroxide | 0.05 | 0.05 | 0.05 |
| Antiseptic | Suitable amount | Suitable amount | Suitable amount |
| Water | Balance | Balance | Balance |

| | | | |
|---|---|---|---|
| (*1) BF Goodrich "Carbopol 980" | | | |

All the hair-styling gels prepared were excellent in conditioning effects such as moist feeling and voluminous feeling after drying for the hair.

### <Preparation of Skin Lotion>

Three kinds of skin lotions having each formulation shown in Table 19 were prepared in the usual manner.

**Table 19:**

| Skin lotion | | | |
|---|---|---|---|
| Ingredient | Example 18 | Example 19 | Example 20 |
| Ingredient A of the invention | | | |
| N^{α}-Lauroyl-L-arginine (of Synthetic Example 1) | 0.1 | | |
| N^{α}-Cocoyl-L-arginine | | 0.1 | |
| N^{α}-Palmitoyl-L-arginine (of Synthetic Example 3) | | | 0.1 |

| Ingredient B of the invention | | | |
|---|---|---|---|
| Propylene glycol | 1.0 | 1.0 | 1.0 |

| Other ingredients | | | |
|---|---|---|---|
| Hydroxypropyl cellulose (*1) | 0.1 | 0.1 | 0.1 |
| Polyoxyethylene(15) glyceryl monostearate | 1.0 | 1.0 | 1.0 |
| Liquid paraffin | 0.2 | 0.2 | 0.2 |
| Antiseptic | Suitable amount | Suitable amount | Suitable amount |
| Water | Balance | Balance | Balance |

| | | | |
|---|---|---|---|
| (*1) Sin-Etsu Chemical Co., Ltd. "HPC" | | | |

All the skin lotions prepared were excellent in feelings upon use such as smoothening property, moist feeling and the like without sticky feeling and occlusive feeling for the skin.

According to the present invention, there can be easily provided cosmetic compositions embodiments of which may be excellent in conditioning effects such as moist feeling and voluminous feeling in the case of hair cosmetics and excellent in feelings upon use such as smoothing property and moist feeling without sticky feeling and occlusive feeling in the case of skin cosmetics.

## Claims

1. A cosmetic composition comprising, as active ingredients, one or more members selected from the group consisting of mono-N^{α}-acylarginines and cosmetic powders whose surfaces have been treated with a mono-N^{α}-acylarginine (Ingredient A) and one or more members selected from the group consisting of polyhydric alcohols (Ingredient B).

2. The cosmetic composition according to Claim 1, wherein said mono-N^{α}-acylarginine is represented by the following general formula (1): 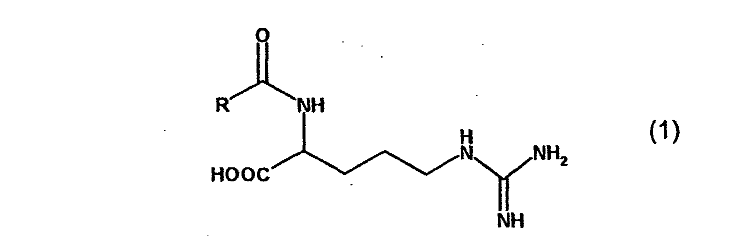 wherein R represents a straight-chain or branched-chain alkyl or alkenyl group having 1-21 carbon atoms.

3. The cosmetic composition according to Claim 1 or 2, wherein said mono-N^{α}-acylarginine is crystals obtainable by neutralizing an acidic or basic solution of a mixed solvent composed of water and one or more members selected from the group consisting of lower alcohols and polyhydric alcohols in which solvent a mono-N^{α}-acylarginine has been dissolved, whereby the mono-N^{α}-acylarginine is crystallized.

4. The cosmetic composition according to Claim 1 or 2, wherein said mono-N^{α}-acylarginine is mono-N^{α}-lauroyl-L-arginine crystals having the physical properties shown in the following Table 1.
**Table 1**
| | |
|---|---|
| (a) | Powder X-ray diffraction peaks (2θ): 12.5°, 21.5°, 21.9°. |
| (b) | Wave numbers of infrared absorption spectra (cm⁻¹): 1684, 1655, 1626, 1545, 1472, 1462, 1447, 1408. |

5. The cosmetic composition according to Claim 1 or 2, wherein said mono-N^{α}-acylarginine is mono-N^{α}-myristoyl-L-arginine crystals having the physical properties shown in the following Table 2.
**Table 2**
| | |
|---|---|
| (a) | Powder X-ray diffraction peaks (2θ): 11.6°, 14.5°, 21.7°, 31.0°, 33.8°. |
| (b) | Wave numbers of infrared absorption spectra (cm⁻¹): 1684, 1655, 1626, 1543, 1472, 1460, 1447, 1408. |

6. The cosmetic composition according to Claim 1 or 2, wherein said mono-N^{α}-acylarginine is mono-N^{α}-palmitoyl-L-arginine crystals having the physical properties shown in the following Table 3.
**Table 3**
| | |
|---|---|
| (a) | Powder X-ray diffraction peaks (2θ) : 10.7°, 13.3°, 21.6°. |
| (b) | Wave numbers of infrared absorption spectra (cm⁻¹): 1684, 1655, 1626, 1543, 1472, 1460, 1448, 1408. |

7. The cosmetic composition according to any one of Claims 1 to 6, wherein said cosmetic composition is a hair cosmetic composition.

8. The cosmetic composition according to any one of Claims 1 to 6, wherein said cosmetic composition is a hair detergent composition.

9. The cosmetic composition according to any one of Claims 1 to 6, wherein said cosmetic composition is a skin cosmetic composition.

10. The cosmetic composition according to any one of Claims 1 to 3 and 7 to 9, wherein said mono-N^{α}-acylarginine is represented by the following general formula (2): 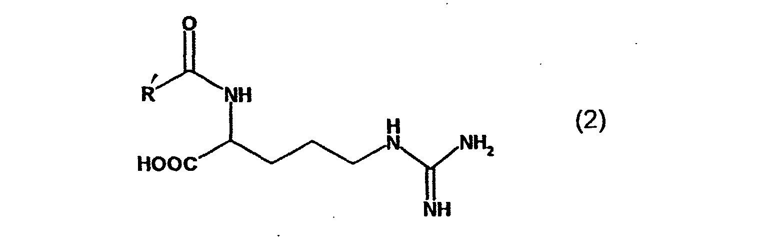 wherein R' represents a straight-chain or branched-chain alkyl or alkenyl group having 11-15 carbon atoms.
